# EUROPEAN PATENT APPLICATION

(11) **EP 1 429 144 A1**
(43) Date of publication of application: **16.06.2004**
(21) Application number: 03257740.5
(22) Date of filing: 09.12.2003
(51) Int. Cl.: G01N 33/532, G01N 33/58

(54) **A precursor for biological probes, uses thereof and methods of its synthesis**

(30) Priority: 09.12.2002 GB 0228648
(71) Applicant: Bio-Med Reagents Limited, Newcastle Upon Tyne, NE2 4AA (GB)
(72) Inventor: Lauc, Gordan, 10000 Zagreb (HR)
(74) Representative: Ouzman, Beverley Nicola Claire

(57) **Abstract**

There is provided a compound comprising a label moiety attached to a linker, the linker having an attachment site adapted for attachment to a ligand, the compound further comprising a photo-reactive group attached to the linker. Such a compound can advantageously be used as a pre-probe for the preparation of bioprobes. Methods of making such pre-probes and bioprobes are also provided.

## Description

### Technical Field

This invention relates to chemical compounds that can be used to produce biochemical probes for the detection and/or isolation of specific molecules from complex biological mixtures.

### Background

The interactions of cell surface receptors, soluble receptors, lectins and adhesion molecules with receptors or other binding partners in complex biological environments are very important for the control and functioning of biological systems. Such interactions participate in many vital physiological processes from fertilisation and development to modulation of the immune system and memory consolidation. Furthermore, such interactions are also closely involved in the pathological mechanisms of many disease processes such as inflammation, cancer, infections and neurological disorders.

The study of these biological interactions and the identification of the components involved are difficult because of the very transient nature of the interactions. Also, any manipulation of complexed receptors results in the breakdown of the complex and the loss of the binding agent.

In co-pending Application GB 2361699A a bioprobe suitable to carry out such investigations is described. Such bioprobes contain:
- a suitable ligand to bind to a biological molecule of interest (for example, an oligosaccharide, peptide, hormone or ganglioside)
- a digoxin moiety;
- a photo-reactive group; and
- a lysine derivative connecting the digoxin moiety, the ligand and the photo-active group.

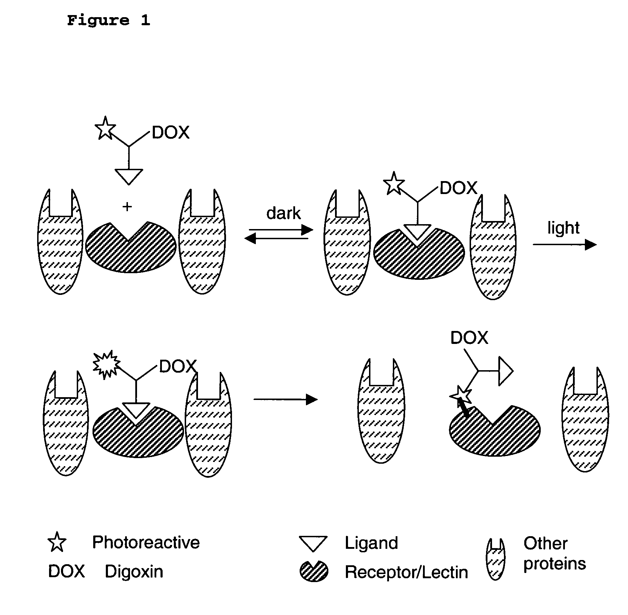

The bioprobe is incubated with a biological sample containing a receptor specific for the ligand. After recognition of the ligand, illuminating the complex with UV light causes formation of a covalent linkage between the photo-active group present on the bioprobe and non-specific site(s) on the binding receptor (see Figure 1). Including digoxin as part of the probe allows the receptor/ligand complex to be easily removed from or identified in a biological mixture using anti-digoxin antibodies. There are many anti-digoxin antibodies available commercially. Furthermore, the high sensitivity of the glycosidic bond to slightly acidic conditions allows the receptor/ligand complex to be easily separated from the remaining part of the probe and thus isolated.

Such bioprobes have very wide application in the understanding of receptors that are involved in biological processes, development of new therapeutic targets and in the diagnosis of many diseases such as cancer, neurodegenerative diseases (e.g. Alzheimer's disease), infertility, inflammatory diseases (e.g. rheumatoid arthritis) and infections.

The above-mentioned co-pending Application GB 2361699A describes a pre-probe which can be provided and used by laboratories to prepare ligand-specific bioprobes and for the isolation and/or characterisation of various ligand-binding bio-macromolecules. These pre-probes are provided to the end-users such as scientific researchers and enable them to prepare bioprobes having desired ligands suitable to study particular receptors. The pre-probe described in Application GB 2361699A however requires two successive synthesising steps to become operational: 1) to covalently bind the ligand to the pre-probe; and 2) to covalently bind the photo-reactive group to the pre-probe. Only then can the bioprobe be used to locate and isolate specific biomolecules which bind to the selected ligand. Such a two-step method of synthesis is cumbersome and time consuming. Also the pre-probe needs to be specifically prepared for each ligand, which increases the cost of the bioprobe.

It has now been found that a pre-probe which overcomes these disadvantages and only requires the addition of the ligand to become functional can be prepared. This pre-probe is stable, effective and can be used to rapidly prepare the above-mentioned bioprobe molecules. Such a flexible approach offers the end user an easy and simple method of designing a wide range of bioprobes and/or bioprobes specific to his need.

### Statement of the invention

The invention relates to a compound comprising, preferably consisting of, a label moiety attached to a linker, the linker having an attachment site adapted for attachment to a ligand, the compound further comprising a photo-reactive group attached to the linker. Such a compound can advantageously be used as a pre-probe for the preparation of bioprobes.

Suitable labels moieties for use in a probe are well known in the art, and the label moiety selected will depend on the method to be used for detecting, separating or visualising the probe when bound to its target molecule. Often the label moiety will be detected and/or separated by use of an antibody or another affinity based system, in which case mention may be made of the biotin/avidin system for example. Alternatively fluorescence may be used as the basis for detection of the probe; in this case mention may be made of green fluorescent protein or yellow fluorescent protein. As a further alternative a radioactive label could be used, and in this case mention may be made of moieties containing ³²P, ¹³¹I, ³⁵S ¹⁴C, ⁴⁵Ca or ³H. These examples of label are included to illustrate the various types of label moieties which are well known in the art and by no means provide an exhaustive list.

It has been found that a particularly efficient pre-probe is obtained when the label moiety comprises a digoxigenin-containing compound, especially digoxin, or a functional analogue thereof.

Preferably the label moiety comprises a glycosidic chain attached to a digoxigenin moiety or a functional equivalent thereof.

In a particularly preferred embodiment the label moiety comprises a digoxin moiety or a functional equivalent thereof.

The term "functional equivalent thereof" specifies that the invention is not restricted to the named molecule alone, but extends to other molecules which achieve the same function as the specific molecule identified. For example, the term includes analogues or mimetics of the named compounds which contain substitutions and/or structural variations. The term particularly encompasses equivalents of digoxigenin or digoxin which would be bound by an antibody raised to an epitope of digoxigenin or digoxin themselves. Modifications to the structure of digoxigenin or digoxin, for example by adding one or more alkyl groups, metal ions or other prosthetic groups or substituents, would be obvious to the person skilled in the art and such equivalents would fall within the scope of the present invention. Exemplary functional equivalents of digoxin would include "digoxin-like" compounds having a steroid structure and a glycosidic chain linked thereto.

Suitably the label moiety is digoxigenin or digoxin preferably digoxin.

Preferably the linker is an amino acid derivative. In one preferred embodiment the linker is a lysine derivative. Preferred alternative linkers include aspartic or glutamic acid derivatives which have an amino group positioned on a side chain.

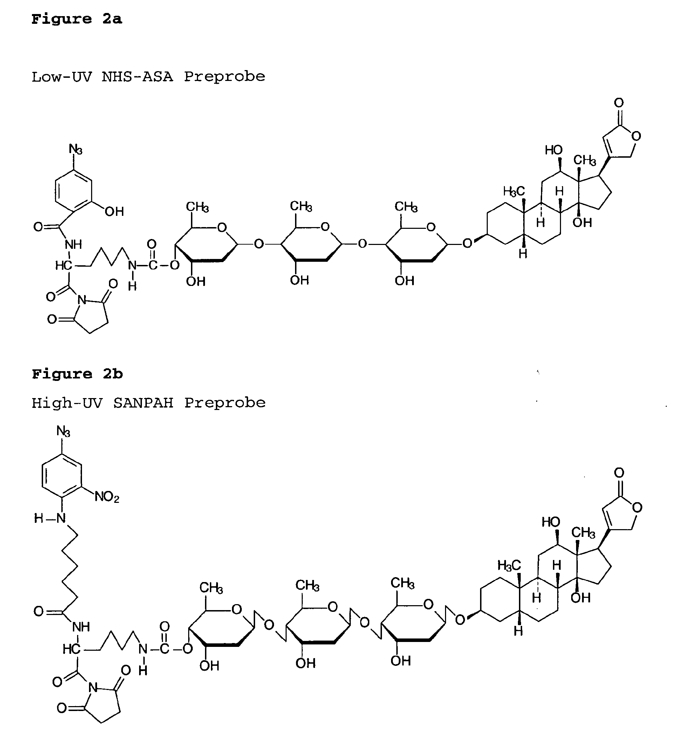

Advantageously, when the label moiety is digoxin, the linker is covalently bound to the extremity of the glycosidic chain of the digoxin moiety which is at the opposite end to the steroid moiety, and advantageously at position 4 of the last digitoxose ring (as shown in Figure 2).

Advantageously, when the label moiety is digoxigenin, the linker is covalently bound to the first ring of the steroid moiety, preferably in position 3.

The attachment site should preferably have a good reactive group. Such a group is chosen to allow easy substitution by a pre-determined ligand. For example, succinimide derivatives such as NHS (N-hydroxysuccinimide) or sulfo-NHS (N-hydroxysulfosuccinimide) or TSTU (N,N,N',N'-Tetramethyl-O-(N-succinimidyl) Uronium Tetrafluoroborate) have been found to be particularly effective groups. Ideally it should easily react with an amino group on the ligand and preferably the amino group should be in a position that does not interfere with the ligand-binding to its specific receptor or binding protein. Such ligands can be, in particular, oligosaccharides, peptides, hormones or gangliosides.

Where the linker is an amino acid, the photo-reactive group is preferably covalently attached to the alpha amino group of the linker. Photo-reactive groups which are able, under irradiation with UV light, to form a covalent bond with a biological macromolecule (like a cellular receptor) are preferred. Examples of such photo-reactive group are 4-azidosalicylic acid or N-(4-azido-2-nitro-phenyl) aminohexanoate.

According to another aspect of the invention there is provided a method of synthesis of a bio-probe which comprises the step of:
- reacting the above defined compound with a ligand under suitable condition to covalently bind said ligand to said attachment site.

Advantageously the attachment site of the compound is provided by an active group such as a succinimide derivative already mentioned above. When the ligand comprises an amino group, the method may further comprise the replacement of said reactive group with said ligand. Preferably the step of covalently binding said ligand to said attachment site is carried out in a single step reaction. Such an aspect of the invention is particularly preferred as it allows a one-step synthesis of suitable bioprobe by end-users.

Advantageously, reaction of the compound with the ligand is carried out in presence of a mixture of DMF and triethylamine. It is further preferred that the ligand and the compound are incubated for at least 24 hours, more preferably at least 36 hours.

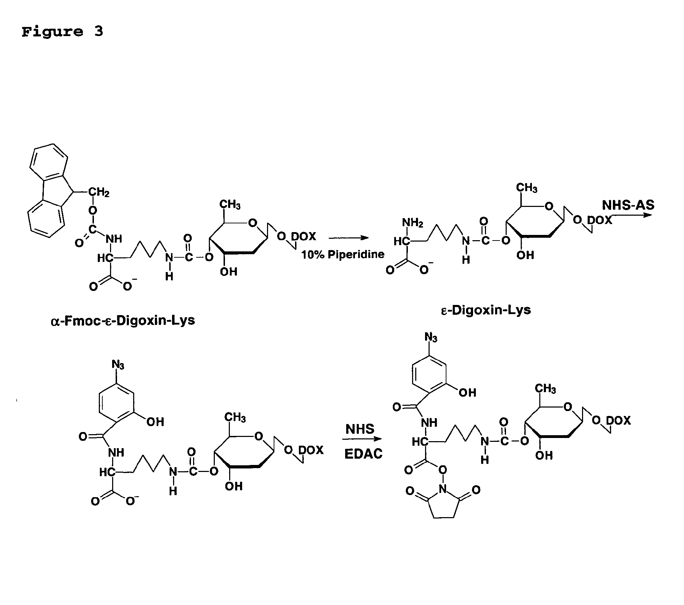

Another aspect of the invention is a method of synthesis of the above defined compound which can be used as a pre-probe. The method comprises the steps of
- providing an α-Fmoc-ε-Digoxin-Lys compound (Figure 3);
- removing the Fmoc moiety by treatment with 10% piperidine;
- covalently attaching the photo-reactive group; and
- carrying out the esterification of the resulting compound with N-hydroxysuccinimide.

The invention also extends to the use of the above compound as a pre-probe for the synthesis of a bioprobe as above mentioned which comprises a digoxin moiety having a glucosidic chain attached to a steroid moiety, a lysine derivative, a ligand attached to the lysine derivative and a photo-reactive group attached to the lysine derivative.

### Brief description of the Figures

Figure 1 is a schematic diagram showing the use of a bio-probe to isolate a specific receptor.
Figures 2a and b show the structure of two pre-probes of the invention. Figure 2a shows a Low-UV NHS-ASA preprobe. Figure 2b shows a High-UV SANPAH preprobe.
Figure 3 shows the synthetic pathway of a pre-probe of the invention.
Figure 4 shows the structure of an exemplary "Man 9" ligand which can be used with the pre-probe of the invention to form a bioprobe.

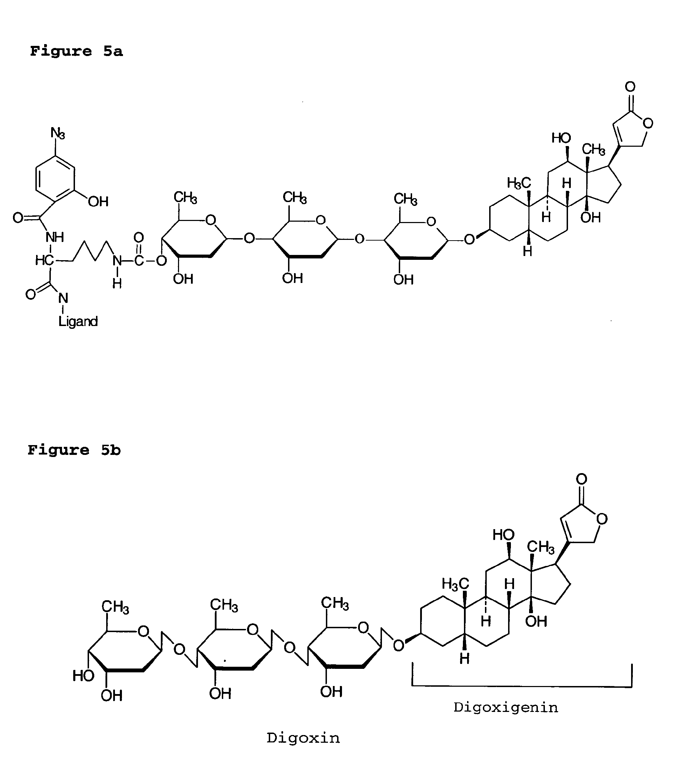

Figure 5a shows the structure of a preferred bioprobe obtained using a pre-probe of the invention.
Figure 5b shows the structure of digoxin and digoxigenin.
Figure 6 shows the electrophoretic separation of the two samples (Lane A no probe and Lane B ligand probe) made according to the method of the invention.

### Examples

### Structure of a "one-step" pre-probe

"One-step" pre-probes are a particularly preferred embodiment of the invention.

The structures of preferred one-step pre-probe molecules are exemplified in Figure 2. They are preferably composed of four components: digoxin, a lysine derivative, succinimide ester and a photo-reactive group. The digoxin part is used to identify or isolate the receptor/ligand complexes. The succinimide ester is used to form the probe by reacting spontaneously with any ligand containing an amino group. The typical photo-active grouping is used to form a covalent bond with a bound receptor after irradiation with UV light. Typical groups are 4-azidosalicylic acid (NHS-ASA activated by exposure to low wavelength UV light) or N-(4-azido-2-nitro-phenyl) aminohexanoate (SANPAH activated by exposure to high wavelength UV light). The lysine derivative is a linkage component in the molecule.

### Synthesis of one-step pre-probe

Synthesis of a one-step pre-probe starts with α-Fmoc-ε-Digoxin-Lys intermediate (shown in Figure 3). This compound can be synthesis following the same procedure described in Patent Application No GB 2361699A.

### Synthesis of α-Fmoc-ε-Digoxin-Lys: Activation of digoxin using CNBr

A 5 M solution of cyanogen bromide (CNBr) in acetonitrile is added to 33 mM digoxin (DOX) in 33% tetrahydrofuran and 66% 2 M K-phosphate buffer (pH = 12) to give 20-fold excess, with constant stirring at room temperature. Formation of the product is monitored by reverse-phase HPLC (30-min) linear gradient 0-50% acetonitrile in water, 0.01% TFA is included in both solvents). The reaction mixture is evaporated to dryness under reduced pressure and the dried powder is redissolved in 10 ml of chloroform and 10 ml of 1 M NaCl. The DOX derivative is extracted into the organic phase and after re-extraction with another 10 ml chloroform, the combined chloroform phases are briefly extracted with 5 ml water and dried under reduced pressure.

### Synthesis of α-Fmoc-ε-Digoxin-Lys: Conjugation of α-Fmoc-lysine with digoxin

A 40 mM solution of α-N-(9-Fluorenylmethoxycarbonyl)-L-lysine (Fmoc-Lys) in 66% tetrahydrofuran, 33% 0.1 M Na-phosphate buffer (pH 7) is added to = 50 µmol of dried activated DOX (100 µmol DOX, molar ratio of α-Fmoc-Lys to DOX 1 to 1, assuming 50% activation and stood at 42°C for 18 - 24 hours with vigorous shaking. The reaction is followed by thin layer chromatography (TLC) using chloroform: methanol: water (v/v) 80:20:1 as a developing solvent. α-Fmoc-Lysine is identified under UV lamp and DOX by "charring" after spraying with 50% H₂SO₄ in 95% ethanol. When ca. 60% of α-Fmoc-lysine (R_{F} = 0.13) is converted to Fmoc-Lys-DOX (R_{F} = 0.52) the reaction is stopped by drying. The dried mixture is dissolved in 10 ml of chloroform and 10 ml of 1% TFA in water containing 1 mmol NaCl and the Fmoc-Lys-DOX is extracted into the organic phase by vigorously shaking for 3 minutes. NaCl (1 mmol) is added to facilitate separation of the phases. The water phase is re-extracted with 2 x 5 ml chloroform and the pooled chloroform solution is slowly added to 18 ml hexane, followed by gentle mixing for 10 minutes. During this time, the precipitate formed consists mainly of Fmoc-DOX-Lys and some DOX. The precipitate and supernatant are separated, and after evaporation, the supernatant is dissolved in 5 ml of chloroform and precipitated with 4.5 ml of hexane. Precipitate from both vials is combined and designated as Fmoc-Lys-DOX.

### Synthesis of a one-step pre-probe

α-Fmoc-ε-Digoxin-Lys is deprotected by removing Fmoc by treatment with 10% piperidine. The photo-reactive cross (PC) linker is then incorporated into the molecule using N-hydroxysuccinimide ester of either 4-azidosalicylic acid or N-(4-azido-2-nitro-phenyl)amino hexanoate. Following purification of PC-Digoxin-Lys, the functional one-step preprobe is prepared by esterification with N-hydroxysuccinimide.

### Incorporation of ligand into one-step preprobe

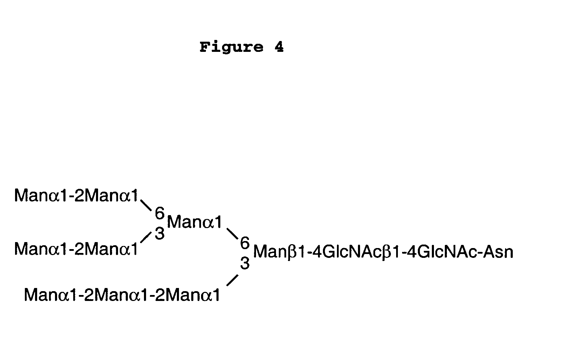

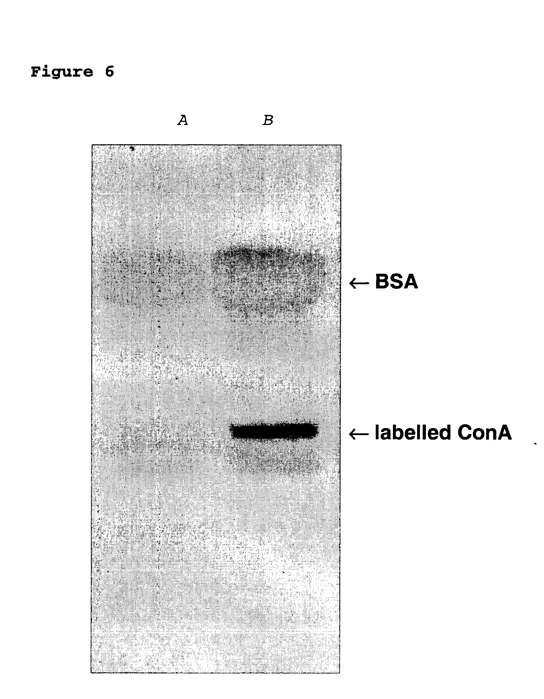

A specific embodiment of these particular one-step pre-probe is the incorporation of a ligand into these structures in a single step. The procedure for doing this will now be described by way of an example incorporating a carbohydrate structure called Man-9 (shown in Figure 4) into the NHS-ASA one-step pre-probe. The presence of the Man9 probe in the molecule is detected by using a specific receptor for it, the lectin Concanavalin A. 10 nmol of Man-9 were dissolved in 20µl of N, N-dimethylformamide (DMF) and added to 100 nmol of the one-step preprobe dissolved in 10µl of DMF and 2.89µl of Triethylamine (1/1000 dilution). This mixture was incubated for 36 h at 20°C and then extracted three times with 350µl of a mixture of water and toluene (6:1). The Man-9 probe was extracted into the aqueous layer and stored at -20°C in the dark. A mixture of Concanavalin A (5µg) and 50µg of Bovine Serum Albumin (BSA) in 5µl phosphate buffered saline (PBS), supplemented with 1 mM Ca²⁺ and Mg²⁺ were added to 0.1 nmol of the Man-9 probe in 10 µl of water and incubated at 37°C in the dark for 120 min. This reaction mixture was irradiated with an UV lamp (low wavelength) for 15 min at 4°C. Concanavalin A and BSA were also incubated with no probe and processed in the same way as the probe but without the Man-9 ligand incorporated. Proteins were reduced, separated by SDS/PAGE and subjected to Western blotting. The probe binding was detected with anti-digoxin antibody labelled with alkaline phosphatase. As shown in Figure 6, the probe only binds to Con A.

These examples are not intended nor should they be construed as limitations on the invention. As one skilled in the art would understand, many variations and modifications may be made in the specific examples provided herein that fall within the spirit and scope of this invention.

## Claims

1. A compound comprising a label moiety attached to a linker, the linker having an attachment site adapted for attachment to a ligand, the compound further comprising a photo-reactive group attached to said linker.

2. The compound of Claim 1 wherein the label moiety comprises a digoxigenin moiety or a functional equivalent thereof.

3. The compound of Claim 1 or 2 wherein the linker is an amino acid derivative.

4. The compound of Claim 3 wherein the amino acid is selected from the group consisting of lysine, aspartic acid and glutamic acid.

5. The compound of Claim 4 wherein the linker is a lysine derivative.

6. The compound of any one of the preceding Claims wherein the linker is covalently attached to the label moiety.

7. The compound of any one of the preceding Claims wherein the label moiety comprises a digoxin moiety or a functional equivalent thereof.

8. The compound of Claim 7 wherein said linker is covalently bound to the extremity of the glycosidic chain of said digoxin moiety or a functional equivalent thereof which is opposite to the steroid moiety of the digoxin moiety or functional equivalent thereof.

9. The compound of Claim 8 wherein the linker is covalently bound at position 4 of a glycosidic ring at said extremity.

10. The compound of any one of the preceding Claims wherein said attachment site has a reactive group.

11. The compound of Claim 10, wherein said reactive group comprises a succinimide derivative.

12. The compound of Claim 11 wherein the group is N-hydroxysuccinimide, N-hydroxysulfosuccinimide or N,N,N',N'-Tetramethyl-O-(N-succinimidyl) Uronium Tetrafluoroborate.

13. The compound of any one of the preceding Claims, wherein said attachment site is adapted for attachment of a ligand which is a biological molecule containing an amino group.

14. The compound of any one of the preceding Claims, wherein said attachment site is adapted for attachment of a ligand which is an oligosaccharide, a peptide, an hormone or a ganglioside.

15. The compound of any one of the preceding Claims, wherein the linker is an amino acid derivative and the photo-reactive group is covalently attached to the alpha amino group of the amino acid.

16. The compound of any one of the preceding Claims, wherein said photo-reactive group is able, under irradiation with UV light, to form a covalent bond with a biological macromolecule.

17. The compound of any one of the preceding Claims wherein said photo-reactive group is 4-azidosalicylic acid or N-(4-azido-2-nitro-phenyl) aminohexanoate.

18. A method of synthesis of a bio-probe which comprises the step of:
- reacting the compound of any one of Claims 1 to 16 with a ligand under suitable conditions to covalently bind said ligand to said attachment site.

19. The method of Claim 18, wherein said attachment site of said compound has a succinimide derivative and wherein said ligand comprises an amino group, said method comprising the replacement of said succinimide derivative with said ligand.

20. The method of Claim 18 or 19, wherein said step of covalently binding said ligand to said attachment site is carried out in a single step reaction.

21. The method of any one of Claims 18 to 20 wherein said ligand is an oligosaccharide, a peptide, a hormone or a ganglioside.

22. The method of any one of Claims 18 to 21, wherein said step of covalently binding said ligand to said attachment site is carried out in presence of a mixture of DMF and triethylamine.

23. The method of Claim 22, wherein said ligand and said compound are incubated for at least 24 hours.

24. A method of synthesis of a compound as claimed in Claim 7, the method comprising the steps of:
- providing an α-Fmoc-ε-Digoxin-Lys compound;
- removing the Fmoc moiety by treatment with 10% piperidine;
- covalently attaching the photo-reactive group; and
- carrying out the esterification of the resulting compound with N-hydroxysuccinimide.

25. Use of the compound of any one of Claims 1 to 17 for the synthesis of a bioprobe which comprises a label moiety comprising digoxigenin or a functional equivalent thereof, a linker, a ligand attached to said linker and a photo-reactive group attached to said linker.
